(19) Europäisches Patentamt / European Patent Office / Office européen des brevets

(11) **EP 3 319 564 B1**

(12) **EUROPEAN PATENT SPECIFICATION**

(45) Date of publication and mention
of the grant of the patent:
**27.11.2019 Bulletin 2019/48**

(21) Application number: **16738384.3**

(22) Date of filing: **06.07.2016**

(51) Int Cl.:
**A61F 9/007** *(2006.01)*

(86) International application number:
**PCT/EP2016/065981**

(87) International publication number:
**WO 2017/005795 (12.01.2017 Gazette 2017/02)**

(54) **OPHTHALMIC SCRAPER DEVICE AND METHOD OF MAKING THE SAME**

OPHTHALMISCHES KRATZGERÄT UND VERFAHREN ZUR DESSEN HERSTELLUNG

DISPOSITIF DE GRATTOIR OPHTALMIQUE ET PROCÉDÉ DE FABRICATION DE CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.07.2015 NL 2015102**

(43) Date of publication of application:
**16.05.2018 Bulletin 2018/20**

(73) Proprietor: **Crea IP B.V.
3311 RN Dordrecht (NL)**

(72) Inventor: **DAM-HUISMAN, Adriaantje Coliene
3208 KM Spijkenisse (NL)**

(74) Representative: **Nederlandsch Octrooibureau
P.O. Box 29720
2502 LS The Hague (NL)**

(56) References cited:
**EP-A1- 0 554 668          US-A- 5 623 942
US-A1- 2014 172 010          US-A1- 2014 277 110**

## Description

## Field of the invention

The present invention relates to an ophthalmic scraper device, in particular an ophthalmic scraper device for ophthalmic surgical assistance. In a further aspect the invention relates to method of making an ophthalmic scraper.

## Prior art

International publication WO 01/19255 discloses an adjustable stiffness membrane scraper usable in ophthalmic applications. A scraping edge projecting beyond a tip of the instrument is provided with abrasive particles.

US patent publication US 5,921,998 discloses an ophthalmic treatment tool having a grip portion, a rod-shaped body attached to the grip portion and an elastic body fitted toward a front end side of the rod-shaped body, wherein a tapered tip of the elastic body is provided with hard inorganic fine-grains or particles.

US patent application US 2007/0282348 discloses an ophthalmic microsurgical instrument for scraping or selectively removing tissue membrane from the retina, or other areas of the eye. The ophthalmic microsurgical instrument is provided with a rod like solid silicone tip having a front most portion which is coated with a plurality of micron size sapphire chips providing an abrasive surface. Both the angle and hardness of the silicone tip are determined for providing maximum tactile feel to a surgeon using the ophthalmic microsurgical instrument.

US patent application US2014/277110 describes a membrane removing forceps including a first forceps jaw having a distal end and a proximal end and a second forceps jaw having a distal end and a proximal end. The membrane removing forceps may include one or more abrasive surfaces configured to raise a portion of a membrane.

The prior art devices thus comprise randomly deposited particles on a tapered tip of a rod shaped body. During use, these particles may be released or come off the instrument and remain in the eye of a patient.

## Summary of the invention

The present invention seeks to provide an improved ophthalmic scraper device, wherein surface material or foreign material of the scraper device is not released and left behind in a patient's eye during a procedure, e.g. an ophthalmic or intraocular procedure.

According to the present invention an ophthalmic scraper device of the type defined in the preamble is provided comprising a handle body at a first end of the ophthalmic scraper device and a flexible rod shaped body at a second end of the ophthalmic scraper device projecting from the handle body, wherein the rod shaped body comprises a flexible tip portion distal to the handle body, the tip portion having an abrasive patterned surface texture, and wherein the rod shaped body, the flexible tip portion and the abrasive patterned surface texture are made of a single flexible material. The flexible material is silicone or a thermoplastic material.

An advantage of the present invention is that the abrasive patterned surface texture is provided as a surface roughness of the single material of the rod shaped body rather than provided by foreign particles bonded, deposited or adhered thereto. As a result the abrasive patterned surface texture of the present invention is a surface roughness feature of the single material of which the rod shaped body is made, thereby avoiding spontaneous release of foreign abrasive particles during use of the scraper device in ophthalmic procedures, such as removing vitreous material from retinal tissue or peeling an internal membrane.

In an embodiment, the tip portion comprises a tapered section provided at least in part with the abrasive patterned surface texture, allowing the tip portion to engage an operation surface at an angle as the ophthalmic scraper device may be manoeuvred at an angle to the operation surface during a procedure.

In an embodiment, the abrasive patterned surface texture is provided on an entire surface of the tip portion. This allows for a maximum scraping surface that maximizes scraping efficiency.

In a further embodiment, the abrasive patterned surface texture is disposed circumferentially around the tip portion along an abrasive length of the rod shaped body, thereby facilitating scraping engagement alongside the flexible rod shaped body when following a curved surface.

In another embodiment, the abrasive patterned surface texture comprises an embossed patterned surface texture, wherein embossed features defining the abrasive patterned surface texture may comprise various ribs, ridges, projections, notches, hairs, polygonal structures and so on. These embossed features may be envisaged as small scale surface deformations of the rod shaped body, in particular the tip portion.

In a more specific embodiment, the abrasive patterned surface texture comprises an alternating pattern of a plurality of parallel grooves and ridges, which is advantageous for increasing scraping action during forward and backward motion of the ophthalmic scraper device.

In an advantageous embodiment, the abrasive patterned surface texture comprises a square or honeycomb pattern of a plurality of recesses, wherein the plurality of recesses are delimited by abrasive walls having relatively sharp edges that provide increased scraping action. The plurality of recesses allow scraped material to be received therein, not only to facilitate removal of scraped material, but also to ensure that the plurality of abrasive walls maintain good scraping contact with a surface to be scraped.

In an even further embodiment, the tip portion is provided with two different surface patterns, e.g. at an upper part and a lower part of the tip portion. This allows a user to have two different scraping actions available during use, by simply turning the ophthalmic scraper device over 180 degrees.

In a further aspect, the present invention seeks to provide an improved method of making an ophthalmic scraper device. According to this further aspect of the invention a method is provided of making an ophthalmic scraper device of the type defined in the preamble, the method comprising providing a rod shaped body of a single, flexible silicone or thermoplastic material, forming a surface area of a tip portion of the rod shaped body for providing an abrasive patterned surface texture on the tip portion, and providing a handle body and connecting the handle body to the rod shaped body at an end thereof opposite the tip portion. This allows to produce the present invention embodiments of an ophthalmic scraper device in a cost-efficient and reproducible manner.

**Short description of drawings**

The present invention will be discussed in more detail hereinafter based on a number of exemplary embodiments with reference to the drawings, in which

Figure 1 shows a side view of an embodiment of an ophthalmic scraper device according to the present invention;
Figure 2 shows a side view of an embodiment of a rod shaped body according to the present invention;
Figure 3 shows side view of an embodiment of a tip portion according to the present invention;
Figure 4 shows a three dimensional view of an embodiment of a ring shaped abrasive patterned surface texture according to the present invention;
Figure 5 shows a three dimensional view of an embodiment of a honeycomb abrasive patterned surface texture according to the present invention, and
Figure 6 shows a side view of an even further embodiment of the ophthalmic scraper device according to the present invention.

**Detailed description of exemplary embodiments**

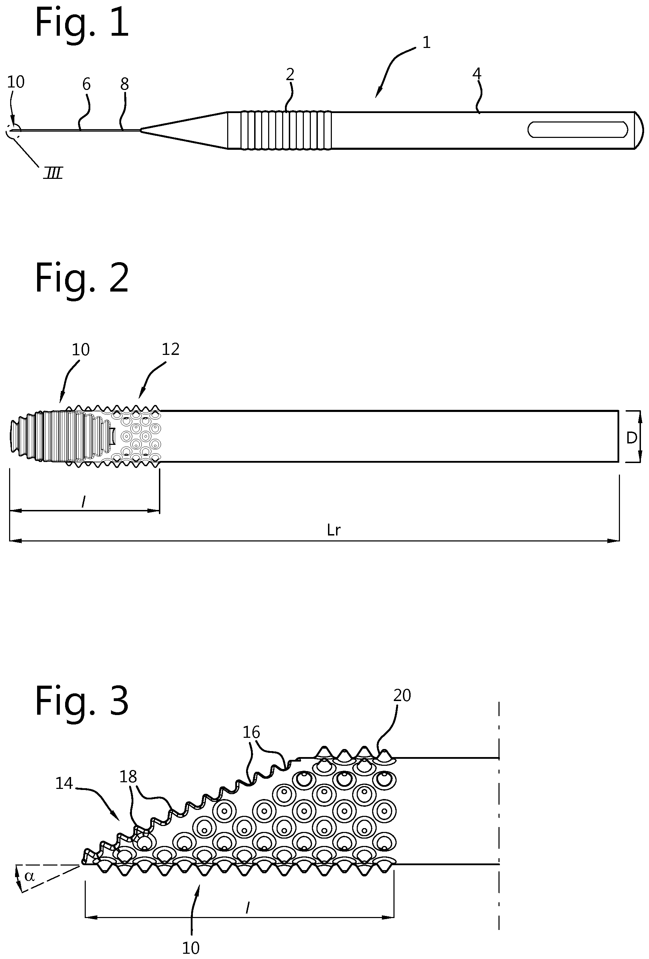

Figure 1 shows a side view of an embodiment of an ophthalmic scraper device 1 according to the present invention. In the embodiment shown, the ophthalmic scraper device 1 comprises a handle body 2 at a first end 4 of the ophthalmic scraper device 1 and a rod shaped body 6 at a second end 8 of the ophthalmic scraper device 1 projecting from the handle body 2. The rod shaped body 6 is made of a single material and comprises a flexible tip portion 10 distal to the handle body 2, wherein the tip portion 10 comprises an abrasive patterned surface texture 12 (not shown in Figure 1). The tip portion 10 is adapted for scraping engagement with tissue to be removed from the eye's surface (e.g. vitreous membrane from the retina) without shedding or releasing surface material of the rod shaped body 6 during e.g. an ophthalmic surgical procedure. The rod shaped body 6 may be integrally formed with the handle body 2, or may be a separate element, e.g. able to be slide into the handle body 2 (when not in use). In a further alternative, the rod shaped body 6 may be attached to an intermediate body, e.g. a capillary tube which is e.g. made of polyimide, which intermediate body is attached inside the handle body 2. The ophthalmic scraper device 1 of the present invention therefore avoids that foreign material is left behind in a patient's eye.

Figure 2 shows a side view of an embodiment of a rod shaped body 6 and Figure 3 shows a side view of an embodiment of a tip portion 10 according to the present invention. These embodiments show the abrasive patterned surface texture 12 as an increased surface roughness for scraping surface material from e.g. the retina of a patient's eye.

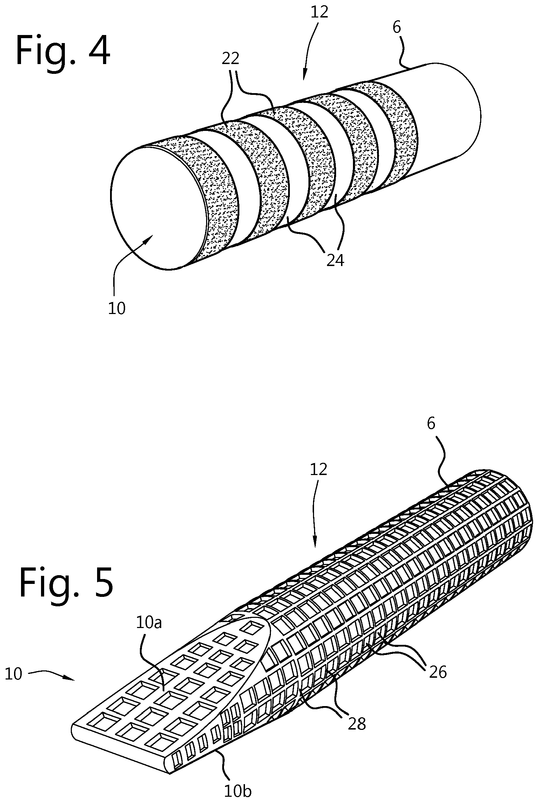

In the embodiments shown in Figure 2 and 3, the tip portion 10 may comprise a tapered section 14 provided at least in part with the abrasive patterned surface texture 12. It will be clear that alternative embodiments can be envisaged having other forms of the tip portion 10, e.g. a rounded tip portion 10 (see embodiment of Figure 4 as described below) or a flattened tip portion 10 having two flat surfaces (see embodiment of Figure 5 below).

The tapered section 14 allows the tip portion 10 to engage a surface, e.g. a retina surface, at an angle as the ophthalmic scraper device 1 may be positioned or manoeuvred at an angle to the surface during a procedure.

In an advantageous embodiment the abrasive patterned surface texture 12 is provided on an entire surface of the tip portion 10, so that a maximum scraping surface is obtained that maximizes scraping efficiency. A taper angle $\alpha$ of the tapered section 14 can be chosen to facilitate a particular scraping action of the ophthalmic scraper device 1. The tapered section 14 e.g. has a taper angle $\alpha$ between 25° and 65° degrees with respect to a longitudinal direction or axis of the rod shaped body 6. It may be envisaged that a user utilizes two or more ophthalmic scraping devices 1, each having a different taper angle $\alpha$ to allow for different engagement angles of the rod shaped body 6 with respect to a tissue surface. In a further embodiment the taper angle $\alpha$ may even be more than 65°, even up to 90°, which would mean that the tip section 10 comprises a straight end surface substantially perpendicular to the longitudinal direction or axis of the rod shaped body 6.

In a further embodiment, the abrasive patterned surface texture 12 is disposed circumferentially around the tip portion 10 along an abrasive length *l* of the rod shaped body 6, thereby allowing scraping engagement alongside the flexible rod shaped body 6 when following a curved surface. Advantageously, increasing the abrasive length *l* along the rod shaped body 6 increases the surface area that can be scraped in a single movement of the ophthalmic scraper device 1. Typically, the (overall) length *Lr* of the rod shaped body 6 is between 0.5 mm to 5 mm in most embodiments. The abrasive length *l* along which the abrasive patterned surface texture 12 can be circumferentially arranged may be any length between zero and the length *Lr* of the rod shaped body 6, depending on scraping requirements of the application.

The rod shaped body 6 may comprise an outer diameter *D* between 0.1 mm to 1.5 mm, which, in combination with the single material of the rod shaped body 6, provides sufficient flexibility of the rod shaped body 6 for delicate scraping requirements during e.g. retina procedures.

In a particular embodiment, the abrasive patterned surface texture 12 comprises a regularly patterned surface texture 12. This embodiment is advantageous for manufacturing the rod shaped body 6, where a regularly patterned surface texture 12 facilitates automated manufacturing.

In light of the present invention, scraping action of the rod shaped body 6 is achieved through the abrasive patterned surface texture 12, which can be viewed as an increased surface roughness of the single material of which the rod shaped body 6 is made. In many embodiments the abrasive patterned surface texture has a surface roughness $R_a$ between 1/1000 mm to 5/10 mm. In this application, surface roughness is a measure in the general terms as used in the art, i.e. a one dimensional parameter defined by the arithmetic average of absolute values y (being a vertical deviation perpendicular to the surface):

$$R_{\mathrm{a}} = \frac{1}{n} \sum_{i=1}^{n} |y_i|$$

The surface texture 12 may have various parameters characterising the surface texture 12, including but not limited to the surface roughness $R_a$. These parameters may be uniform for the entire surface texture 12, or areas may exist within the surface texture 12 with different parameter values. It would also be possible to have a non-uniform surface texture 12, e.g. a gradually increasing or decreasing surface roughness Ra.

The increased surface roughness may be embodied as raised or sunken surface features applied to the single material of the rod shaped body 6. Such raised and sunken surface features of the rod shaped body 6, tip portion 10 and/or tapered section 14 will be referred to "embossments", thus wherein the abrasive patterned surface texture 12 comprises an embossed abrasive patterned surface texture 12. The embossments may comprise a plurality of ribs, ridges, projections, notches, hairs, various polygonal (e.g. hexagonal) structures and so on.

In Figure 3 an exemplary embodiment is shown where the abrasive patterned surface texture 12 of a part of the tip portion 10 is formed by a plurality of notches 20. These embossments representing the abrasive patterned surface texture 12 need not be regularly arranged but may also be irregular or randomly arranged across surfaces of the rod shaped body 6, the tip portion 10 and/or the tapered section 14 thereof. The abrasive patterned surface structure 12 may be a three-dimensional or intentionally formed structure as part of the tip portion 10.

As depicted in the embodiment of Figure 3, the abrasive patterned surface texture 12 on the tapered section 14 may comprise an alternating pattern of a plurality of parallel grooves 16 and ridges 18. The alternating pattern of a plurality of parallel grooves 16 and ridges 18 are arranged substantially perpendicular to the longitudinal direction of axis of the rod shaped body 6. This increases scraping action during forward and backward motion of the ophthalmic scraper device 1. As shown, the tapered section 14 of the tip portion 10 may comprise such an alternating pattern of parallel grooves 16 and ridges 18. This allows for an improved scraping action of the tapered section 14 during forward and backward motion when the rod shaped body 6 is positioned at an angle associated with the taper angle $\alpha$ of the tapered section 14.

Figure 4 shows a detailed view of an exemplary embodiment of a ring shaped abrasive patterned surface texture 12 on the tip portion 10 of the rod shaped body 6, wherein the tip portion 10 has a rounded end surface. In the embodiment shown, the abrasive patterned surface texture 12 comprises a plurality of abrasive rings 22 circumferentially disposed around the rod shaped body 6. Each abrasive ring 22 has a higher surface roughness than surrounding surface areas 24 of the rod shaped body 6. This ring shaped pattern allows enhanced scraping action in longitudinal direction of the rod shaped body 6.

Figure 5 depicts an exemplary embodiment wherein the abrasive patterned surface texture 12 comprises a square or honeycomb pattern of a plurality of recesses 26. This square or honeycomb pattern provides a relative high surface roughness due to the plurality of abrasive walls 28 delimiting the plurality of recesses 26. These abrasive walls 28 may comprise relative sharp edges that facilitate scraping and wherein the plurality of recesses 26 allow scraped material to be temporality received therein to improve removal of scraped debris. This square or honeycomb pattern provides substantially uniform scraping action irrespective of the applied direction of motion of the rod shaped body 6. The tip portion 10 of the rod shaped body 6 in this embodiment has a flattened end with a top surface 10a and a bottom surface 10b. As noted above, the surface texture 12 may be uniform or non-uniform, e.g. the top surface 10a having a higher roughness $R_a$ than the bottom surface 10b.

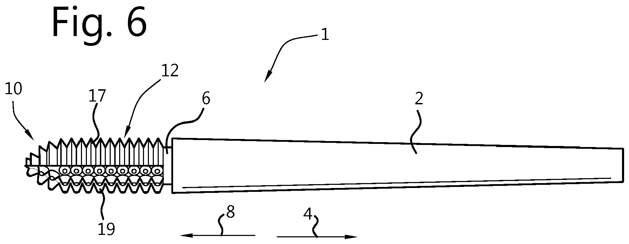

In Figure 6, a side view is shown of an even further embodiment of the ophthalmic scraper device 1 according to the present invention. Again, a handle body 2 is provided at a first end 4 of the ophthalmic scraper device 1, and a rod shaped body 6 is provided at a second end 8. The distal part of the rod shaped body 6 has a tip portion 10 which is provided with an surface texture 12. In this embodiment, the tip portion 10 is provided with two different surface patterns on two half-dome parts of the tip portion 10. The upper part (as drawn in Figure 6) is provided with a surface texture comprising ridges 17 directed perpendicular to the longitudinal direction of the ophthalmic scraper device 1, whereas the lower part is provided with a surface texture comprising cone shaped extensions 19 (in a regular pattern). This allows to have an availability to the user of two different types of scraping action using a single ophthalmic scraper device 1, depending on which half-dome part is directed towards the eye during use. As shown in the embodiment of Figure 6, the extending parts 17, 19 forming the abrasive pattern of the surface texture 12 may change in form towards the tapering end of tip portion 10 (e.g. getting a slighter sharper edge and smaller dimensions near the end of the tip portion 10).

It is noted that one or more of the various embodiments described with reference to Figures 1-6 may also be combined in a single ophthalmic scraper device 1.

In alternative embodiments the abrasive patterned surface texture 12 may also comprise other patterns comprising e.g. polygon shaped recesses 26, e.g. triangular, hexagonal recesses, wherein the abrasive walls 28 thereof are positioned at different angles, thereby providing alternative scraping action for particular directions of motion of the rod shaped body 6.

As mentioned earlier, the rod shaped body 6 can be made of a single material having at least in part an abrasive patterned surface texture 12 applied thereto. The single material may be any medical grade silicone or therpoplastic material, e.g. silicone rubber or thermoplastic elastomer (TPE). In order to provide a sufficient flexibility of at least the tip portion 10, yet still provide sufficient strength to act as a scraper device, the material is selected to have a certain hardness, e.g. a Shore A value between 0 and 75.

If the rod shaped body 6 is made of a flexible material, it may be advantageous to arrange the rod shaped body 6 slidably within the handle body 2. This is e.g. achieved by providing a capillary tube or sleeve of e.g. polyimide material (which is stiffer than the flexible material of the rod shaped body 6) around the rod shaped body 6. The capillary tube is mounted slidable within a second capillary tube (e.g. from a metal material) which is fixed inside the handle body 2 with an external actuator element (slider handle). The rod shaped body 6 can then be extended using the slider handle.

To further increase scraping capabilities of the rod shaped body 6, alternative embodiments may be provided wherein the single material is a resin, optionally further comprising an additive in the form of particles, e.g. sand, diamond dust etc. This embodiment allows for an additional, higher surface roughness if so required.

In a further aspect the invention relates to a method of making an ophthalmic scraper device. Reference is made to the Figures 1 to 6.

According to the present invention the rod shaped body 6 is to be provided at least in part with an increased surface roughness to allow for scraping engagement with a surface, e.g. a retina surface, wherein the surface roughness is to be provided as part of the single material of which the rod shaped body 6 is made. To the end the method comprises the step of providing a rod shaped body 6 of a single, flexible material, wherein the material is silicone or a thermoplastic material. The method then comprises the step of forming a surface area of a tip portion 10 of the rod shaped body 6 for providing an abrasive patterned surface texture 12 on the tip portion 10. The tip portion 10 may also comprise a circumferential outer surface area along an abrasive length $l$ of the rod shaped body 6 in a longitudinal direction or axis thereof.

As a last step, the method further comprises providing a handle body 2 and connecting the handle body 2 to the rod shaped body 6 at an end thereof opposite the tip portion 10.

The method of the present invention is advantageous in that the abrasive patterned surface texture 12 is part of the material of which the rod shaped body 6 is made and that no foreign abrasive particles are used to form an abrasive surface texture. As a result, the ophthalmic scarper device 1 manufactured according to the present invention does not release particles that could stay in the patient's eye after e.g. a retinal procedure, which further reduces the risk of possible damage to the eye.

In an embodiment the tip portion 10, or the entire rod shaped body 6, is formed using an injection moulding process, wherein the abrasive patterned surface texture 12 is provided through an injection mould inner surface texture. Such an injection moulding process is efficient and capable of high reproducibility in providing surface textures in one single step. In an alternative embodiment, the surface texture 12 is applied as a separate step, e.g. using pressure or compression moulding techniques.

In an alternative embodiment the tip portion 10, or the entire rod shaped body 6, is formed using an extrusion process. In such an extrusion process the abrasive patterned surface texture 12 may comprises a plurality of grooves and ridges in longitudinal direction of the rod shaped body 6.

So in further embodiments of the method, forming a surface area of a tip portion 10 of the rod shaped body 6 for providing an abrasive patterned surface texture 12 on the tip portion 10 may comprise an injection moulding process and/or extrusion process, possibly in one single step.

In further advantageous embodiments, forming the abrasive patterned surface texture 12 of the tip portion 10 is obtained by laser treatment. In this way surfaces areas of the rod shaped body 6, in particular the tip portion 10, are subjected to a laser treatment for altering the surface roughness of the single, flexible material of which the rod shaped body 6 is made. The entire rod shaped body 6 may be subjected to such a laser treatment as well in specific embodiments.

In an embodiment, the laser treatment comprises a laser ablation or laser melting process for obtaining the abrasive patterned surface texture 12. In this embodiment a surface area of the rod shaped body 6 is transformed and/or removed such that the surface roughness thereof is increased, e.g. between 1/1000 mm to 5/10 mm. One or more surface areas of the rod shaped body 6 that are not subjected to the laser treatment retain a smoother surface roughness.

In addition to utilizing a radiation source, e.g. laser source, for obtaining the abrasive patterned surface texture 12 as outlined above, it also possible that in an embodiment the method comprises the step of mechanically embossing the tip portion 10 or rod shaped body 6 through a rolling process, e.g. utilizing an embossing roll or embossing plate to mechanically impress embossments comprising raised and/or sunken areas within the single, flexible material of the rod shaped body 6. Such a mechanical embossing process is also capable of providing regular and/or random arrangements of surface recesses and/or projections within and on the rod shaped body 6 for increasing at least in part the surface roughness thereof.

The embodiments of the present invention have been described above with reference to a number of exemplary embodiments as shown in and described with reference to the drawings. Modifications and alternative implementations of some parts or elements are possible, and are included in the scope of protection as defined in the appended claims.

## Claims

1. Ophthalmic scraper device comprising a handle body (2) at a first end (4) of the ophthalmic scraper device (1) and a rod shaped body (6) at a second end (8) of the ophthalmic scraper device (1) projecting from the handle body (2), wherein the rod shaped body (6) comprises a flexible tip portion (10) distal to the handle body (2),
the tip portion (10) having an abrasive patterned surface texture (12), and
**characterised in that** the rod shaped body (6), the flexible tip portion (10) and the abrasive patterned surface texture (12) are made of a single flexible material, wherein the material is silicone or a thermoplastic material.

2. Ophthalmic scraper device according to claim 1, wherein the tip portion (10) comprises a tapered section (14) provided at least in part with the abrasive patterned surface texture (12).

3. Ophthalmic scraper device according claim 1 or 2, wherein the abrasive patterned surface texture (12) is provided on an entire surface of the tip portion (10).

4. Ophthalmic scraper device according any one of claims 1 to 3, wherein the abrasive patterned surface texture (12) is disposed circumferentially around the tip portion (10) along an abrasive length (*l*) of the rod shaped body (6).

5. Ophthalmic scraper device according to any one of claims 1 to 4, wherein the abrasive patterned surface texture (12) comprises a regularly patterned surface texture, embossed surface texture, or an alternating pattern of a plurality of parallel grooves and ridges.

6. Ophthalmic scraper device according to any one of claims 1 to 5, wherein the abrasive patterned surface texture (12) comprises a square or honeycomb pattern (26, 28) of a plurality of recesses.

7. Ophthalmic scraper device according to any one of claims 1 to 6, wherein the single material is a resin, optionally further comprising an additive in the form of particles.

8. Ophthalmic scraper device according to any one of claims 1 to 7, wherein the rod shaped body (6) comprises an outer diameter between 0.1 mm to 1.5 mm.

9. Ophthalmic scraper device according to any one of claims 1 to 8, wherein the rod shaped body (6) has a length (*Lr*) between 0.5 mm to 5 mm.

10. Ophthalmic scraper device according to any one of claims 1 to 9, wherein the patterned surface texture (12) has a surface roughness Ra between 1/1000 mm to 5/10 mm.

11. Ophthalmic scraper device according to any one of claims 1 to 10, wherein the tip portion (10) is provided with two different surface patterns (12).

12. Method of making an ophthalmic scraper device for ophthalmic surgical assistance, comprising
providing a rod shaped body (6) of a single, flexible material, wherein the material is silicone or a thermoplastic material;
forming a surface area of a tip portion (10) of the rod shaped body (6) for providing an abrasive patterned surface texture (12) on the tip portion (10); and
providing a handle body (4) and connecting the handle body (4) to the rod shaped body (6) at an end thereof opposite the tip portion (10).

13. Method according to claim 12, wherein the rod shaped body (6) with the tip portion (10) is formed using an injection moulding and/or extrusion process.

**14.** Method according to claim 12 or 13, wherein the abrasive patterned surface texture (12) of the tip portion (10) is obtained by laser treatment.

**15.** Method according to claim 14, wherein the laser treatment comprises a laser ablation or laser melting process.


**Patentansprüche**

**1.** Ophthalmische Schabvorrichtung, die einen Griffkörper (2) an einem ersten Ende (4) der ophthalmische Schabvorrichtung (1) und einen stabförmigen Körper (6) an einem zweiten Ende (8) der ophthalmische Schabvorrichtung (1) aufweist, der aus dem Griffkörper (2) vorsteht, wobei

der stabförmige Körper (6) entfernt von dem Griffkörper (2) einen elastischen Vorderabschnitt (10) aufweist,
der Vorderabschnitt (10) eine abrasiv gemusterte Oberflächenstruktur (12) hat, und sich dadurch auszeichnet, dass
der stabförmige Körper (6), der elastische Vorderabschnitt (10) und die abrasiv gemusterte Oberflächenstruktur (12) aus einem einzigen elastischen Material hergestellt sind, wobei das Material Silikon oder ein thermoplastisches Material ist.

**2.** Ophthalmische Schabvorrichtung nach Anspruch 1, wobei der Vorderabschnitt (10) einen verjüngten Bereich (14) aufweist, der mindestens teilweise mit der abrasiv gemusterten Oberflächenstruktur (12) versehen ist.

**3.** Ophthalmische Schabvorrichtung nach Anspruch 1 oder 2, wobei die abrasiv gemusterte Oberflächenstruktur (12) auf der gesamten Fläche des Vorderabschnittes (10) vorgesehen ist.

**4.** Ophthalmische Schabvorrichtung nach einem der Ansprüche 1 bis 3, wobei die abrasiv gemusterte Oberflächenstruktur (12) umlaufend um den Vorderabschnitt (10) entlang einer Abrasivlänge (*l*) des stabförmigen Körpers (6) angeordnet ist.

**5.** Ophthalmische Schabvorrichtung nach einem der Ansprüche 1 bis 4, wobei die abrasiv gemusterte Oberflächenstruktur (12) eine regelmäßige gemusterte Oberflächenstruktur, eine geprägte Oberflächenstruktur oder ein wechselndes Muster von einer Vielzahl an parallelen Nuten und Rippen aufweist.

**6.** Ophthalmische Schabvorrichtung nach einem der Ansprüche 1 bis 5, wobei die abrasiv gemusterte Oberflächenstruktur (12) ein rechteckiges oder ein wabenförmiges Muster (26, 28) einer Vielzahl an Vertiefungen aufweist.

**7.** Ophthalmische Schabvorrichtung nach einem der Ansprüche 1 bis 6, wobei das einzige Material ein Harz ist, das alternativ zusätzlich ein Additiv in Form von Partikeln aufweist.

**8.** Ophthalmische Schabvorrichtung nach einem der Ansprüche 1 bis 7, wobei der stabförmige Körper (6) einen Außendurchmesser zwischen 0.1 mm und 1.5 mm aufweist.

**9.** Ophthalmische Schabvorrichtung nach einem der Ansprüche 1 bis 8, wobei der stabförmige Körper (6) eine Länge (Lr) zwischen 0.5 und 5 mm aufweist.

**10.** Ophthalmische Schabvorrichtung nach einem der Ansprüche 1 bis 9, wobei die gemusterte Oberflächenstruktur (12) eine Oberflächenrauhigkeit Ra zwischen 1/1000 mm und 5/10 mm aufweist.

**11.** Ophthalmische Schabvorrichtung nach einem der Ansprüche 1 bis 10, wobei der Vorderabschnitt (10) mit zwei verschiedenen Oberflächenmustern (12) versehen ist.

**12.** Verfahren für die Herstellung einer Ophthalmische Schabvorrichtung zur augenchirurgischen Unterstützung, das besteht aus

Bereitstellen eines stabförmigen Körpers (6) aus einem einzigen, elastischen Material, wobei das Material Silikon oder ein thermoplastisches Material ist;
Formen eines flächigen Bereiches eines Vorderabschnittes (10) aus dem stangeförmigen Körpers (6), um eine abrasiv gemusterte Oberflächenstruktur (12) auf dem Vorderabschnitt (10) bereitzustellen; und
Bereitstellen eines Griffkörpers (4) und Verbinden des Griffkörpers (4) mit dem stabförmigen Körper (6) an dessen einem Ende gegenüber des Vorderabschnittes (10).

**13.** Verfahren nach Anspruch 12, wobei der stabförmige Körper (6) mit dem Vorderabschnitt (10) geformt ist, indem ein Spritzguss- und/oder ein Strangpressverfahren verwendet wird.

**14.** Verfahren nach Anspruch 12 oder 13, wobei die abrasiv gemusterte Oberflächenstruktur (12) auf dem Vorderabschnitt (10) durch Laserbearbeitung erwirkt wird.

**15.** Verfahren nach Anspruch 14, wobei die Laserbearbeitung ein Laserablations- oder Laserschmelzverfahren aufweist.

## Revendications

**1.** Dispositif de raclage ophtalmique comprenant un corps de poignée (2) à une première extrémité (4) du dispositif de raclage ophtalmique (1) et un corps en forme de tige (6) à une seconde extrémité (8) du dispositif de raclage ophtalmique (1) faisant saillie du corps de poignée (2), le corps en forme de tige (6) comprenant une partie d'embout flexible (10) en position distale par rapport au corps de poignée (2), la partie d'embout (10) ayant une texture de surface à motif abrasif (12),
et **caractérisé en ce que**
le corps en forme de tige (6), la partie d'embout flexible (10) et la texture de surface à motifs abrasifs (12) sont constitués d'un seul matériau flexible, le matériau consistant en silicone ou en un matériau thermoplastique.

**2.** Dispositif de raclage ophtalmique selon la revendication 1, dans lequel la partie d'embout (10) comprend une section effilée (14) munie au moins en partie de la texture de surface à motifs abrasifs (12).

**3.** Dispositif de raclage ophtalmique selon la revendication 1 ou 2, dans lequel la texture de surface à motifs abrasifs (12) est pourvue sur toute la surface de la partie d'embout (10).

**4.** Dispositif de raclage ophtalmique selon l'une quelconque des revendications 1 à 3, dans lequel la texture de surface à motifs abrasifs (12) est disposée de manière circonférentielle autour de la partie d'embout (10) le long d'une longueur abrasive (1) du corps en forme de tige (6).

**5.** Dispositif de raclage ophtalmique selon l'une quelconque des revendications 1 à 4, dans lequel la texture de surface à motifs abrasifs (12) comprend une texture de surface à motifs réguliers, une texture de surface gaufrée ou un motif d'une pluralité de rainures et de nervures parallèles alternées.

**6.** Dispositif de raclage ophtalmique selon l'une quelconque des revendications 1 à 5, dans lequel la texture de surface à motifs abrasifs (12) comprend un motif carré ou un motif en nid d'abeille (26, 28) d'une pluralité d'évidements.

**7.** Dispositif de raclage ophtalmique selon l'une quelconque des revendications 1 à 6, dans lequel le matériau seul est une résine, comprenant éventuellement en outre un additif sous la forme de particules

**8.** Dispositif de raclage ophtalmique selon l'une quelconque des revendications 1 à 7, dans lequel le corps en forme de tige (6) présente un diamètre extérieur compris entre 0,1 mm et 1,5 mm.

**9.** Dispositif de raclage ophtalmique selon l'une quelconque des revendications 1 à 8, dans lequel le corps en forme de tige (6) a une longueur (*Lr*) comprise entre 0,5 mm et 5 mm.

**10.** Dispositif de raclage ophtalmique selon l'une quelconque des revendications 1 à 9, dans lequel la texture de surface à motif (12) a une rugosité de surface Ra comprise entre 1/1000 mm et 5/10 mm.

**11.** Dispositif de raclage ophtalmique selon l'une quelconque des revendications 1 à 10, dans lequel la partie d'embout (10) est munie de deux motifs de surface différents (12).

**12.** Procédé de fabrication d'un dispositif de raclage ophtalmique pour une assistance chirurgicale ophtalmique, comprenant

- la fourniture d'un corps en forme de tige (6) formé d'un seul matériau flexible, dans lequel le matériau est du silicone ou un matériau thermoplastique ;
- la formation d'une surface d'une partie d'embout (10) du corps en forme de tige (6) pour assurer une texture de surface à motifs abrasifs (12) sur la partie d'embout (10) ; et
- la fourniture d'un corps de poignée (4) et relier le corps de poignée (4) à une extrémité du corps en forme de tige (6) opposée à la partie d'embout (10).

**13.** Procédé selon la revendication 12, dans lequel le corps en forme de tige (6) avec la partie d'embout (10) est formé par utilisation d'un procédé de moulage par injection et / ou d'extrusion.

**14.** Procédé selon la revendication 12 ou 13, dans lequel la texture de surface à motifs abrasifs (12) de la partie d'embout (10) est obtenue par traitement au laser.

**15.** Procédé selon la revendication 14, dans lequel le traitement au laser comprend un processus d'ablation au laser ou de fusion au laser.

# Fig. 1

# Fig. 2

# Fig. 3

Fig. 4

Fig. 5

# Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0119255 A
- US 5921998 A
- US 20070282348 A
- US 2014277110 A